# EUROPÄISCHE PATENTSCHRIFT

(11) **EP 0 703 225 B1**
(45) Veröffentlichungstag und Bekanntmachung des Hinweises auf die Patenterteilung: **03.09.1997**
(21) Anmeldenummer: 95114214.0
(22) Anmeldetag: 11.09.1995
(51) Int. Cl.: C07D 249/12

(54) **Verfahren zur Herstellung von Alkoxytriazolinonen**
Process for the preparation of alkoxytriazolinones
Procédé pour la préparation d'alcoxytriazolinones

(30) Priorität: 23.09.1994 DE 4433969
(43) Veröffentlichungstag der Anmeldung: 27.03.1996
(73) Patentinhaber: BAYER AG, 51368 Leverkusen (DE)
(72) Erfinder: Wroblowsky, Heinz-Jürgen, Dr., D-40764 Langenfeld (DE); König, Klaus, Dr., D-51519 Odenthal (DE)

(56) Entgegenhaltungen:
- EP-A- 0 477 646
- EP-A- 0 507 171

## Beschreibung

Die Erfindung betrifft ein neues, auch technisch durchführbares Verfahren zur Herstellung von weitgehend bekannten Alkoxytriazolinonen, welche als Zwischenprodukte zur Herstellung von agrochemischen Wirkstoffen verwendet werden können.

Alkoxytriazolinone und mehrere Methoden zu ihrer Herstellung sind bereits bekannt (vgl. J. Indian Chem. Soc. 6 (1929), 565-575; J. Chem. Soc. Perkin I 1973, 2644-2646; Arch. Pharm. 307 (1974), 889-891; EP-A 477646; EP-A 507171). Nach diesen bekannten Synthesemethoden werden Alkoxytriazolinone jedoch nur in sehr unbefriedigenden Ausbeuten erhalten.

Ferner ist bekannt, daß durch Methylierung von Urazol oder 4-Methylurazol mit Diazomethan (CH₂N₂) 5-Methoxy-4-methyl-2,4-dihydro-3H-1,2,4-triazol-3-on gebildet wird [vergl. F. Arndt et al, Rev. Fac. Sci. Istanbul 13A, S. 127 bis 144 (1948)]; diese Methode liefert zwar das Triazolinon in hoher Ausbeute, kann aber technisch nicht durchgeführt werden.

Es wurde nun gefunden, daß man Alkoxytriazolinone der allgemeinen Formel (I) 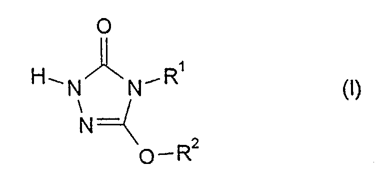 in welcher
- R¹: für jeweils gegebenenfalls substituiertes Alkyl, Alkenyl, Alkinyl, Cycloalkyl, Cycloalkylalkyl, Aryl oder Arylalkyl steht und
- R²: für jeweils gegebenenfalls substituiertes Alkyl, Alkenyl, Alkinyl, Cycloalkyl, Cycloalkylalkyl, Aryl oder Arylalkyl steht,
in sehr guten Ausbeuten und in hoher Reinheit erhält, wenn man
Iminokohlensäurediester der allgemeinen Formel (II) 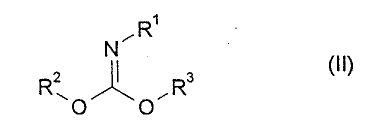 in welcher
- R¹ und R²: die oben angegebenen Bedeutungen haben und
- R³: für jeweils gegebenenfalls substituiertes Alkyl, Aryl oder Arylalkyl steht,
mit Carbazinsäureestern der allgemeinen Formel (III) 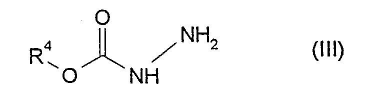 in welcher
- R⁴: für jeweils gegebenenfalls substituiertes Alkyl, Aryl oder Arylalkyl steht,
gegebenenfalls in Gegenwart eines Reaktionshilfsmittels und gegebenenfalls in Gegenwart eines Verdünnungsmittels bei Temperaturen zwischen -20°C und +120°C umsetzt ("erste Umsetzungsstufe") und die hierbei gebildeten Semicarbazid-derivate der allgemeinen Formel (IV) 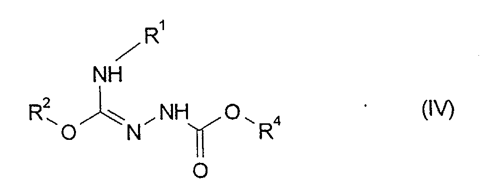 in welcher
- R¹, R² und R⁴: die oben angegebenen Bedeutungen haben,
- und/oder die entsprechenden hierzu tautomeren Verbindungen -,
gegebenenfalls nach Zwischenisolierung, in Gegenwart einer Base und gegebenenfalls in Gegenwart eines Verdünnungsmittels bei Temperaturen zwischen 20°C und 100°C cyclisierend kondensiert ("zweite Umsetzungsstufe").

Überraschenderweise können die Alkoxytriazolinone der allgemeinen Formel (I) nach dem erfindungsgemäßen Verfahren in erheblich höheren Ausbeuten als nach den meisten bekannten Synthesemethoden erhalten werden.

Gegenüber der "Diazomethan-Methode" (F. Arndt et al, 1. c.) hat das erfindungsgemäße Verfahren den entscheidenden Vorteil, daß es auch technisch durchführbar ist.

Im Gegensatz zum bekannten Verfahren, welches bei höheren Temperaturen durchzuführen ist und bei welchem Phenol als Koppelprodukt entsteht - R⁴ steht für Phenyl (vgl. EP-A 507171, Beispiele II-1 und II-2) - kann das erfindungsgemäße Verfahren auch problemlos unter Abspaltung von einfachen Alkanolen durchgeführt werden, die wesentlich einfacher und mit geringerem Energieaufwand als Phenol zurückgewonnen werden können.

In vielen Fällen können vorteilhaft im Gegensatz zum bekannten Stand der Technik (vgl. EP-A 507171) auch "unsymmetrische" Iminokohlensäure-diester (R² und R³ haben dann verschiedene Bedeutung, wobei OR³ eine bessere Abgangsgruppe als OR² ist) als Ausgangsstoffe eingesetzt werden. Als sehr gute Komponenten R³ in den Abgangsgruppen OR³ seinen beispielsweise Methyl, Ethyl, Phenyl, Benzyl, Methoxyethyl und Ethoxyethyl genannt.

Das erfindungsgemäße Verfahren stellt somit eine wertvolle Bereicherung des Standes der Technik dar.

Die Erfindung betrifft vorzugsweise die Herstellung von Verbindungen der Formel (I), in welcher
- R¹: für jeweils gegebenenfalls durch Halogen oder C₁-C₄-Alkoxy substituiertes Alkyl, Alkenyl oder Alkinyl mit jeweils bis zu 6 Kohlenstoffatomen, für jeweils gegebenenfalls durch Halogen oder C₁-C₄-Alkyl substituiertes Cycloalkyl oder Cycloalkylalkyl mit jeweils 3 bis 6 Kohlenstoffatomen im Cycloalkylteil und gegebenenfalls 1 bis 4 Kohlenstoffatomen im Alkylteil, oder für jeweils gegebenenfalls durch Carboxy, Cyano, Nitro, Halogen, C₁-C₄-Alkyl, C₁-C₄-Halogenalkyl, C₁-C₄-Alkoxy, C₁-C₄-Halogenalkoxy oder C₁-C₄-Alkoxy-carbonyl substituiertes Aryl oder Arylalkyl mit jeweils 6 oder 10 Kohlenstoffatomen im Arylteil und gegebenenfalls 1 bis 4 Kohlenstoffatomen im Alkylteil steht und
- R²: für jeweils gegebenenfalls durch Halogen oder C₁-C₄-Alkoxy substituiertes Alkyl, Alkenyl oder Alkinyl mit jeweils bis zu 6 Kohlenstoffatomen, für jeweils gegebenenfalls durch Halogen oder C₁-C₄-Alkyl substituiertes Cycloalkyl oder Cycloalkylalkyl mit jeweils 3 bis 6 Kohlenstoffatomen im Cycloalkylteil und gegebenenfalls 1 bis 4 Kohlenstoffatomen im Alkylteil, oder für jeweils gegebenenfalls durch Carboxy, Cyano, Nitro, Halogen, C₁-C₄-Alkyl, C₁-C₄-Halogenalkyl, C₁-C₄-Alkoxy, C₁-C₄-Halogenalkoxy oder C₁-C₄-Alkoxy-carbonyl substituiertes Aryl oder Arylalkyl mit jeweils 6 oder 10 Kohlenstoffatomen im Arylteil und gegebenenfalls 1 bis 4 Kohlenstoffatomen im Alkylteil steht.

Die Erfindung betrifft insbesondere die Herstellung von Verbindungen der Formel (I), in welcher
- R¹: für jeweils gegebenenfalls durch Fluor, Chlor und/oder Brom, Methoxy oder Ethoxy substituiertes Methyl, Ethyl, n- oder i-Propyl, n-, i-, s- oder t-Butyl, für jeweils gegebenenfalls durch Fluor, Chlor und/oder Brom substituiertes Propenyl, Butenyl, Propinyl oder Butinyl, für jeweils gegebenenfalls durch Fluor, Chlor, Brom, Methyl oder Ethyl substituiertes Cyclopropyl, Cyclobutyl oder Cyclopropylmethyl, oder für jeweils gegebenenfalls durch Carboxy, Cyano, Fluor, Chlor, Brom, Methyl, Ethyl, Trifluormethyl, Methoxy, Ethoxy, Difluormethoxy, Trifluormethoxy, Methoxycarbonyl oder Ethoxycarbonyl substituiertes Phenyl oder Benzyl steht und
- R²: für jeweils gegebenenfalls durch Fluor, Chlor und/oder Brom, Methoxy oder Ethoxy substituiertes Methyl, Ethyl, n- oder i-Propyl, n-, i-, s- oder t-Butyl, für jeweils gegebenenfalls durch Fluor, Chlor und/oder Brom substituiertes Propenyl, Butenyl, Propinyl oder Butinyl, für jeweils gegebenenfalls durch Fluor, Chlor, Brom, Methyl oder Ethyl substituiertes Cyclopropyl, Cyclobutyl oder Cyclopropylmethyl, oder für jeweils gegebenenfalls durch Carboxy, Cyano, Fluor, Chlor, Brom, Methyl, Ethyl, Trifluormethyl, Methoxy, Ethoxy, Difluormethoxy, Trifluormethoxy, Methoxycarbonyl oder Ethoxycarbonyl substituiertes Phenyl oder Benzyl steht.

Verwendet man beispielsweise Methylimino-kohlensäure-O-(2-ethoxy-ethylester)-O-n-propylester und Carbazinsäure-ethylester als Ausgangsstoffe, so kann der Reaktionsablauf beim erfindungsgemäßen Verfahren durch das folgende Formelschema skizziert werden:

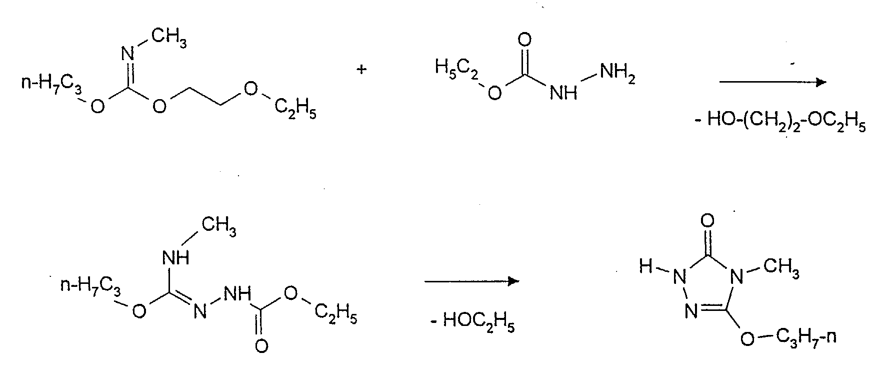

Die beim erfindungsgemäßen Verfahren zur Herstellung der Verbindungen der allgemeinen Formel (I) als Ausgangsstoffe zu verwendenden Iminokohlensäurediester sind durch die Formel (II) allgemein definiert. In der Formel (II) haben R¹ und R² vorzugsweise bzw. insbesondere diejenigen Bedeutungen, die bereits oben im Zusammenhang mit der Beschreibung der Verbindungen der Formel (I) als bevorzugt bzw. als insbesondere bevorzugt für R¹ und R² angegeben wurden; R³ steht vorzugsweise für jeweils gegebenenfalls durch C₁-C₄-Alkoxy, Phenoxy oder Benzyloxy substituiertes C₁-C₄-Alkyl, Phenyl oder Benzyl, insbesondere für Methyl, Ethyl, Propyl, Cyclopropyl, Methoxyethyl oder Ethoxyethyl.

Die Ausgangsstoffe der Formel (II) sind bekannt und/oder können nach an sich bekannten Verfahren hergestellt werden (vgl. Chem. Ber. 120 (1987), 339-344; Herstellungsbeispiele).

Die beim erfindungsgemäßen Verfahren weiter als Ausgangsstoffe zu verwendenden Carbazinsäureester sind durch die Formel (III) allgemein definiert. In der Formel (III) steht R⁴ vorzugsweise für jeweils gegebenenfalls durch C₁-C₄-Alkoxy, Phenoxy oder Benzyloxy substituiertes C₁-C₄-Alkyl, Phenyl oder Benzyl, insbesondere für Methyl, Ethyl, Propyl, Methoxyethyl oder Ethoxyethyl.

Die Ausgangsstoffe der Formel (III) sind bekannte organische Synthesechemikalien.

Als Verdünnungsmittel zur Durchführung des erfindungsgemäßen Verfahrens kommen (in den beiden Umsetzungsstufen) die üblichen organischen Lösungsmittel in Betracht. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Chloroform, Tetrachlormethan; Ether, wie Diethylether, Diisopropylether, Dioxan, Tetrahydrofuran oder Ethylenglykoldimethyl- oder -diethylether; Ketone, wie Aceton, Butanon oder Methyl-isobutyl-keton; Nitrile, wie Acetonitril, Propionitril oder Benzonitril; Amide, wie N,N-Dimethylformamid, N,N-Dimethylacetamid, N-Methylformanilid, N-Methyl-pyrrolidon oder methylphosphorsäuretriamid; Ester wie Essigsäuremethylester oder Essigsäureethylester; Sulfoxide, wie Dimethylsulfoxid; Alkohole, wie Methanol, Ethanol, n- oder i-Propanol, n-, i-, s- oder t-Butanol, Ethylenglykolmonomethylether, Ethylenglykolmonoethylether, Diethylenglykolmonomethylether, Diethylenglykolmonoethylether; deren Gemische mit Wasser oder reines Wasser.

Alkohole, wie Methanol, Ethanol, n- oder i-Propanol werden als Verdünnungsmittel besonders bevorzugt.

Die erfindungsgemäßen Umsetzungen können jedoch gegebenenfalls auch ohne Verwendung von Verdünnungsmitteln durchgeführt werden.

Das erfindungsgemäße Verfahren wird in der ersten Stufe vorzugsweise in Gegenwart eines geeigneten Reaktionshilfsmittels durchgeführt. Als solche kommen vorzugsweise Protonensäuren, wie z.B. Salzsäure, Schwefelsäure, Phosphorsäure, Kohlensäure, Essigsäure, Propionsäure, Pivalinsäure, Methansulfonsäure, Benzoesäure, Benzolsulfonsäure und p-Toluolsulfonsäure - gegebenenfalls auch polymere Säuren oder saure Ionenaustauscher - in Betracht.

Pivalinsäure, Essigsäure und (wässrige) Salzsäure werden als Reaktionshilfsmittel bei der ersten Stufe des erfindungsgemäßen Verfahrens besonders bevorzugt.

Das erfindungsgemäße Verfahren wird in der zweiten Stufe in Gegenwart einer Base durchgeführt. Es kommen hierbei alle üblichen anorganischen oder organischen Basen in Betracht. Hierzu gehören beispielsweise Erdalkali- oder Alkalimetallhydride, -hydroxide, -amide, -alkoholate, -acetate, -carbonate oder -hydrogencarbonate, wie beispielsweise Natriumhydrid, Natriumamid, Natriummethylat, Natriumethylat, Kalium-tert.-butylat, Natriumhydroxid, Kaliumhydroxid, Ammoniumhydroxid, Natriumacetat, Kaliumacetat, Calciumacetat, Ammoniumacetat, Natriumcarbonat, Kaliumcarbonat, Kaliumhydrogencarbonat, hydrogencarbonat oder Ammoniumcarbonat sowie basische organische Stickstoffverbindungen, wie Trimethylamin, Triethylamin, Tributylamin, N,N-Dimethylanilin, N,N-Dimethyl-benzylamin, Pyridin, N-Methylpiperidin, N,N-Dimethylaminopyridin, 5-Ethyl-2-methyl-pyridin, Diazabicyclooctan (DABCO), bicyclononen (DBN) oder Diazabicycloundecen (DBU).

Alkalimetallalkoholate, wie Natriummethylat oder Natriumethylat, und Alkalimetallhydroxide, wie Natrium- oder Kaliumhydroxid - jeweils gegebenenfalls im entsprechenden Alkohol bzw. in Wasser gelöst - werden als Basen bei der zweiten Stufe des erfindungsgemäßen Verfahrens besonders bevorzugt.

Die Reaktionstemperaturen können bei der Durchführung der ersten Stufe des erfindungsgemäßen Verfahrens in einem größeren Bereich variiert werden. Im allgemeinen arbeitet man bei Temperaturen zwischen -20°C und 120°C, vorzugsweise bei Temperaturen zwischen 0°C und 100°C, insbesondere bei Temperaturen zwischen 20°C und 80°C.

Die Reaktionstemperaturen können bei der Durchführung der zweiten Stufe des erfindungsgemäßen Verfahrens in einem größeren Bereich variiert werden. Im allgemeinen arbeitet man bei Temperaturen zwischen 20°C und 100°C, vorzugsweise bei Temperaturen zwischen 30°C und 90°C, insbesondere bei Temperaturen zwischen 40°C und 80°C.

Das erfindungsgemäße Verfahren wird in beiden Stufen im allgeineinen unter Normaldruck durchgeführt. Es ist jedoch auch möglich, unter erhöhtem oder vermindertem Druck - im allgemeinen zwischen 0,1 bar und 10 bar - zu arbeiten.

Zur Durchführung des erfindungsgemäßen Verfahrens zur Herstellung der Verbindungen der Formel (I) setzt man pro Mol Iminokohlensäurediester der Formel (II) im allgemeinen 0,5 bis 1,2 Mol, vorzugsweise 0,8 bis 1,1 Mol Carbazinsäureester der Formel (III) und gegebenenfalls 0,001 bis 2,0 Mol, vorzugsweise 0,01 bis 1,0 Mol Reaktionshilfsmittel ein.

In einer bevorzugten Ausführungsform des erfindungsgemäßen Verfahrens werden die Ausgangsstoffe der Formel (II) und der Formel (III) sowie gegebenenfalls ein Reaktionshilfsmittel in einem geeigneten Verdünnungsmittel vermischt und bei der erforderlichen Temperatur gerührt, bis praktisch kein Ausgangsmaterial mehr vorhanden ist. Die Isolierung des Zwischenproduktes der Formel (IV) kann dann auf übliche Weise, beispielsweise durch Einengen, Digerieren des Rückstandes mit einem organischen Lösungsmittel, wie z.B. Methyl-t-butylether, und Absaugen durchgeführt werden. Das Zwischenprodukt der Formel (IV) kann aber auch ohne Zwischenisolierung mit einer Base - gegebenenfalls in einem der oben angegebenen Verdünnungsmittel gelöst - versetzt und bei der zur cyclisierenden Kondensation erforderlichen Temperatur bis zum Ende der Umsetzung gerührt werden.

Die Aufarbeitung zur Isolierung der Produkte der Formel (I) kann nach üblichen Methoden durchgeführt werden. Beispielsweise wird eingeengt, in Wasser aufgenommen und - z.B. mit Salzsäure - neutralisiert oder angesäuert. Sofern das Produkt hierbei kristallin anfällt, wird es durch Absaugen isoliert. Andernfalls wird mit einem mit Wasser praktisch nicht mischbaren organischen Lösungsmittel, wie z.B. Essigsäureethylester, geschüttelt, die organische Phase - z.B. mit Magnesiumsulfat - getrocknet und filtriert. Nach sorgfältigem Abdestillieren des Lösungsmittels unter vermindertem Druck wird dann das Produkt der Formel (I) als Rückstand erhalten.

Die so erhältlichen Rohprodukte können durch Umkristallisation, Verrühren mit einem geeigneten organischen Lösungsmittel, wie z.B. Petrolether, oder durch Destillation gereinigt werden.

Die nach dem erfindungsgemäßen Verfahren herzustellenden Verbindungen der Formel (I) können als Zwischenprodukte zur Herstellung von herbizid wirksamen Verbindungen verwendet werden (vgl. EP-A 477646 und EP-A 507171).

### Herstellungsbeispiele:

### Beispiel 1

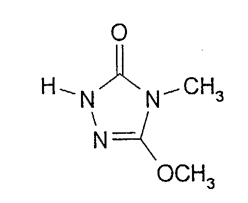

Eine Mischung aus 208 g (2,0 Mol) Carbazinsäure-ethylester, 206 g (2,0 Mol) Methyliminokohlensäure-dimethylester und 500 ml Methanol wird 12 Stunden bei 50°C und weitere 3 Stunden bei 70°C gerührt. Dann werden bei 40°C 360 g einer 30%igen Lösung von Natriummethanolat in Methanol (2,0 Mol NaOCH₃) dazu gegeben und die Reaktionsmischung wird 3 Stunden bei 50°C gerührt. Unter Eiskühlung wird dann mit 20%iger wässriger Salzsäure neutralisiert, anschließend das Methanol weitgehend abdestilliert und die Restmenge mit Wasser auf ca. 500 ml aufgefüllt. Nach mehrstündigem Stehenlassen bei 5°C wird das kristallin angefallene Rohprodukt (243 g) durch Absaugen isoliert. Es enthält neben 6,7% Wasser noch etwas Kochsalz und organische Verunreinigungen. Zur Reinigung wird es in 750 ml Toluol aufgenommen, durch azeotrope Destillation entwässert und heiß filtriert. Das Filtrat wird abgekühlt und das kristalline Produkt durch Absaugen isoliert.

Man erhält 207 g (80% der Theorie) 5-Methoxy-4-methyl-2,4-dihydro-3H-1,2,4-triazol-3-on vom Schmelzpunkt 148°C.

### Beispiel 2

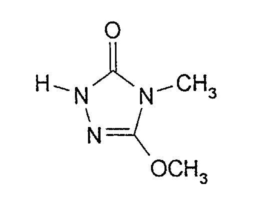

Eine Mischung aus 208 g (2,0 Mol) Carbazinsäure-ethylester, 206 g (2,0 Mol) Methyliminokohlensäure-dimethylester, 4,0 g (0,04 Mol) Pivalinsäure und 1250 ml Methanol wird 3 Tage bei 20°C gerührt. Dann werden bei 40°C 360 g einer 30%igen Lösung von Natriummethanolat in Methanol (2,0 Mol NaOCH₃) dazu gegeben und die Reaktionsmischung wird 3 Stunden bei 50°C gerührt. Anschließend wird mit 20%iger Salzsäure neutralisiert und die weitere Aufarbeitung wie bei Beispiel 1 beschrieben durchgeführt.

Man erhält 220 g (85% der Theorie) 5-Methoxy-4-methyl-2,4-dihydro-3H-1,2,4-triazol-3-on vom Schmelzpunkt 148°C.

### Beispiel 3

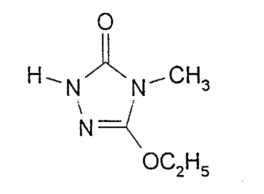

Eine Mischung aus 1740 g (16,56 Mol) Carbazinsäure-ethylester, 2169 g (16,56 Mol) Methyliminokohlensäure-diethylester und 2 Liter Ethanol wird 16 Stunden bei 50°C und weitere 8 Stunden bei 80°C gerührt. Dann werden bei 50°C 2982 g einer 30%igen Lösung von Natriummethanolat in Methanol (16,56 Mol NaOCH₃) tropfenweise dazu gegeben und die Reaktionsmischung wird 3 Stunden bei 50°C gerührt. Unter Eiskühlung wird dann mit 30%iger wässriger Salzsäure neutralisiert und anschließend unter vermindertem Druck eingeengt. Der Rückstand wird in 2,5 Liter auf 80°C erwärmtem N,N-Dimethyl-formamid aufgenommen und durch Absaugen vom Kochsalz befreit. Das Filtrat wird eingeengt, das als Rückstand erhaltene Rohprodukt durch Vakuumdestillation gereinigt und nach Erstarren aus Toluol umkristallisiert.

Man erhält 2019 g (89% der Theorie) 5-Ethoxy-4-methyl-2,4-dihydro-3H-1,2,4-triazol-3-on vom Schmelzpunkt 126°C.

### Beispiel 4

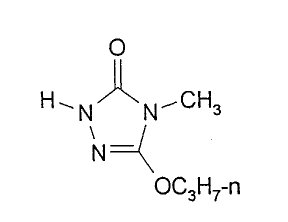

Eine Mischung aus 630 g (6,0 Mol) Carbazinsäure-ethylester, 954 g (6,0 Mol) Methyliminokohlensäure-dipropylester und 600 ml n-Propanol wird bei 0°C unter Kühlen mit einer Eis-Kochsalz-Mischung mit einer Lösung von 612 g (6,0 Mol) Pivalinsäure in 200 ml n-Propanol tropfenweise versetzt und die komplette Mischung wird 30 Minuten bei 0°C bis 10°C gerührt. Dann werden 2169 g einer 30%igen Lösung von Natriummethanolat in Methanol (12 Mol NaOCH₃) dazu gegeben und die Reaktionsmischung wird 3 Stunden bei 50°C gerührt. Unter Eiskühlung wird dann mit konz. Salzsäure neutralisiert, anschließend durch Absaugen vom Kochsalz befreit und das Filtrat eingeengt. Das als Rückstand erhaltene Rohprodukt wird durch Vakuumdestillation gereinigt.

Man erhält 808 g (83% der Theorie) 4-Methyl-5-propoxy-2,4-dihydro-3H-1,2,4-triazol-3-on als wachsartiges Produkt.

Schmelzpunkt: 74°C (aus Aceton).

### Beispiel 5

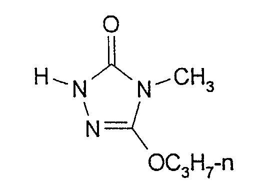

Eine Mischung aus 104 g (1,0 Mol) Carbazinsäure-ethylester, 159 g (1,0 Mol) Methyliminokohlensäure-dipropylester und 200 ml n-Propanol wird unter Eiskühlung mit einer Lösung von 20,4 g (0,2 Mol) Pivalinsäure in 10 ml n-Propanol tropfenweise versetzt und die komplette Mischung wird ohne Kühlung 30 Minuten gerührt. Dann werden 108 g einer 30%igen Lösung von Natriummethanolat in Methanol (0,6 Mol NaOCH₃) dazu gegeben und die Reaktionsmischung wird 3 Stunden bei 50°C und weitere 4 Stunden bei 60°C bis 80°C gerührt. Unter Eiskühlung wird dann mit konz. Salzsäure neutralisiert und anschließend unter vermindertem Druck eingeengt. Das im Rückstand verbliebene Rohprodukt wird durch Vakuumdestillation gereinigt.

Man erhält 137 g (82,5% der Theorie) 4-Methyl-5-propoxy-2,4-dihydro-3H-1,2,4-triazol-3-on als wachsartiges Produkt.

Schmelzpunkt: 74°C (aus Aceton).

### Beispiel 6

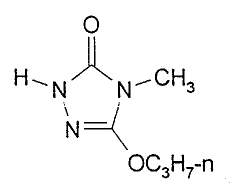

25 g (123 mMol) N'-(α-Methylamino-α-propoxy-methylen)-hydrazin-N-carbonsäure-ethylester werden in 200 ml Methanol vorgelegt und bei 5°C bis 10°C werden 23,2 g einer Lösung von Natriummethanolat (127 mMol NaOCH₃) in Methanol tropfenweise dazu gegeben. Die Mischung wird 6 Stunden bei 50°C gerührt und dann im Wasserstrahlvakuum eingeengt. Der Rückstand wird in 100 ml Wasser aufgenommen und unter Eiskühlung mit konz. Salzsäure angesäuert. Nach Sättigen der Lösung mit Kochsalz wird fünfmal mit Essigsäureethylester extrahiert. Die vereinigten Extraktionslösungen werden mit Magnesiumsulfat getrocknet und filtriert. Vom Filtrat wird das Lösungsmittel unter vermindertem Druck sorgfältig abdestilliert.

Man erhält 17,75 g (88,5% der Theorie) 4-Methyl-5-propoxy-2,4-dihydro-3H-1,2,4-triazol-3-on als wachsartiges Produkt.

Schmelzpunkt: 74°C (aus Aceton).

### Beispiel 7

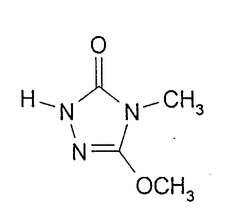

14 g (80 mMol) N'-(α-Methylamino-α-methoxy-methylen)-hydrazin-N-carbonsäure-ethylester werden in 120 ml Methanol vorgelegt, dann werden 7,6 g einer Lösung von Natriumhydroxid (86 mMol NaOH) in Wasser tropfenweise dazu gegeben. Die Mischung wird 5 Stunden bei 50°C gerührt und dann im Wasserstrahlvakuum eingeengt. Der Rückstand wird mit 20%iger wässriger Salzsäure angesäuert und das hierbei kristallin angefallene Produkt durch Absaugen isoliert.

Man erhält 11,3 g 5-Methoxy-4-methyl-2,4-dihydro-3H-1,2,4-triazol-3-on (73%ig, Rest: Natriumchlorid); Ausbeute: 80% der Theorie.

### Beispiel 8

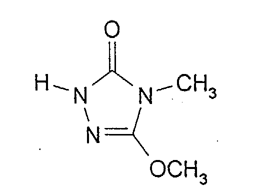

14 g (80 mMol) N'-(α-Methylamino-α-methoxy-methylen)-hydrazin-N-carbonsäure-ethylester werden in 120 ml Methanol vorgelegt und bei 10°C werden 15,2 g einer Lösung von Natriummethanolat (84 mMol NaOCH₃) in Methanol tropfenweise dazu gegeben. Die Mischung wird 6 Stunden bei 50°C gerührt und dann im Wasserstrahlvakuum eingeengt. Der Rückstand wird in 50 ml einer gesättigten wässrigen Kochsalzlösung aufgenommen und unter Eiskühlung mit 12%iger Salzsäure angesäuert. Das hierbei kristallin angefallenene Produkt wird durch Absaugen isoliert.

Man erhält 11,2 g 5-Methoxy-4-methyl-2,4-dihydro-3H-1,2,4-triazol-3-on (91%ig, Rest: Natriumchlorid); Ausbeute: 99% der Theorie.

### Beispiel 9

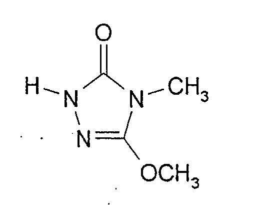

83,8 g (0,794 Mol) Carbazinsäure-ethylester werden in 500 ml Methanol vorgelegt, dann werden 88 g (0,833 Mol) Methyliminokohlensäure-dimethylester zugetropft und 0,95 g (0,016 Mol) Essigsäure dazu gegeben. Die Mischung wird 15 Stunden und nach Zugabe von weiteren 4,2 g (0,04 Mol) Methyliminokohlensäuredimethylester und 0,48 g (0,008 Mol) Essigsäure weitere 6 Stunden bei 20°C gerührt. Dann läßt man 74,7 g 45%ige wässrige Natronlauge (0,84 Mol NaOH) zulaufen und erhitzt die Mischung 6 Stunden auf 55°C bis 58°C. Anschließend wird das Lösungsmittel im Wasserstrahlvakuum entfernt, der Rückstand in 220 ml Eiswasser aufgenommen und unter Eiskühlung durch Zugabe von konz. Salzsäure der pH-Wert auf ca. 6 eingestellt. Das hierbei kristallin angefallene Produkt wird durch Absaugen isoliert.

Man erhält 104 g (85% der Theorie) 5-Methoxy-4-methyl-2,4-dihydro-3H-1,2,4-triazol-3-on (86,8%ig, Rest: Natriumchlorid und Wasser).

### Beispiel 10

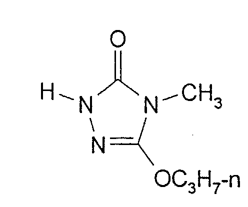

Eine Mischung aus 147,4 g (1,4 Mol) Carbazinsäure-ethylester, 265 g (1,4 Mol) Methyliminokohlensäure-O-(2-ethoxyethylester)-O-n-propylester und 150 ml Propanol wird 10 Stunden auf 80°C und weitere 2 Stunden auf 100°C erhitzt. Nach Abkühlen auf 50°C werden innerhalb von 30 Minuten 252 g einer 30%igen Lösung von Natriummethanolat in Methanol (1,4 Mol NaOCH₃) eindosiert. Nach 3 Stunden Rühren bei 50°C wird unter Kühlen mit Eis/Kochsalz durch Zugabe von 138 g konz. Salzsäure (1,4 Mol HCl) neutralisiert. Dann werden im Wasserstrahlvakuum bei einer Sumpftemperatur von 80°C Methanol, Propanol, Ethoxyethanol und Wasser weitgehend abdestilliert. Das als Rückstand verbliebene Rohprodukt wird durch Vakuumdestillation über eine auf 80°C aufgeheizte Brücke isoliert.

Man erhält 208 g 4-Methyl-5-propoxy-2,4-dihydro-3H-1,2,4-triazol-3-on (90,1%ig, Ausbeute: 85,5% der Theorie), welches durch Umkristallisation aus Toluol/Cyclohexan (1:1) rein erhalten werden kann.

Schmelzpunkt: 74°C.

### Beispiel 11

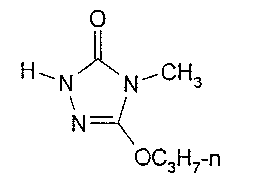

Eine Mischung aus 147,4 g (1,4 Mol) Carbazinsäure-ethylester, 265 g (1,4 Mol) Methyliminokohlensäure-O-(2-ethoxyethylester)-O-n-propylester und 200 ml Propanol wird unter Eiskühlung mit 3,6 g Pivalinsäure versetzt und eine Stunde bei 20°C gerührt. Dann werden weitere 3,6 g Pivalinsäure dazu gegeben und der Rührer wird abgestellt. Nach weiteren 2 Stunden bei 20°C ist der Ansatz durchkristallisiert. Durch Erwärmen auf 50°C entsteht wieder eine homogene Mischung. Innerhalb von 30 Minuten werden dann 252 g einer 30%igen Lösung von Natriummethanolat in Methanol (1,4 Mol NaOCH₃) eindosiert. Nach 3 Stunden Rühren bei 50°C wird unter Kühlen mit Eis/Kochsalz durch Zugabe von 138 g konz. Salzsäure (1,4 Mol HCl) neutralisiert. Dann werden im Wasserstrahlvakuum bei einer Sumpftemperatur von 80°C Methanol, Propanol, Ethoxyethanol und Wasser weitgehend abdestilliert. Das als Rückstand verbliebene Rohprodukt wird durch Vakuumdestillation über eine auf 80°C aufgeheizte Brücke isoliert.

Man erhält 216 g 4-Methyl-5-propoxy-2,4-dihydro-3H-1,2,4-triazol-3-on (90,35%ig, Ausbeute: 90% der Theorie), welches durch Umkristallisation aus Toluol/Cyclohexan (1:1) rein erhalten werden kann.

Schmelzpunkt: 74°C.

### Beispiel 12

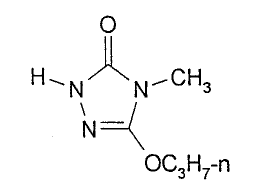

309 g (1,5 Mol) Bis-(2-ethoxyethyl)-carbonat werden unter Kühlung mit einem Wasserbad mit 78,75 g (1,575 Mol) Hydrazinhydrat vermischt und 16 Stunden bei Raumtemperatur gerührt. Nach einer weiteren Stunde bei 50°C werden Hydratwasser, Hydrazinüberschuß und ein Teil des gebildeten Ethoxyethanols bei 15 mbar abgezogen (zusammen 91 g).

Als Rückstand erhält man Carbazinsäure-(2-ethoxyethylester) der Formel H₅C₂O-CH₂CH₂-O-CO-NH-NH₂. Nun werden darin unter Kaltwasser-Kühlung 238,5 g (1,5 Mol) Methylimino-kohlensäure-dipropylester und eine katalytische Menge (1,5 g) Pivalinsäure eingerührt; nach 2 Stunden wird nochmals die gleiche Menge (1,5 g) Pivalinsäure zugegeben. Nach 12 Stunden bei Raumtemperatur wird noch 1 Stunde bei 70°C gerührt, dann mit 58,4 g einer 30 %igen Lösung von Natriummethanolat in Methanol (0,325 Mol NaOCH₃) versetzt und 7 Stunden bei 70°C gerührt.

Anschließend wird durch Zugabe von 32 g konz. Salzsäure (37 %ig) unter Eiskühlung neutralisiert (pH-Kontrolle). Nach dem Abdestillieren aller flüchtigen Bestandteile bis 130°C / 15 mbar wird das gewünschte Produkt aus dem Rückstand unter weiter vermindertem Druck (mittels Ölpumpe) abdestilliert und hierdurch vom gebildeten Natriumchlorid abgetrennt.

Man erhält 221 g Destillat mit einem Gehalt von 95,8 % an 4-Methyl-5-propoxy-2,4-dihydro-3H-1,2,4-triazol-3-on. Unter Berücksichtigung der Analysenproben entspricht dies einer Ausbeute von 93 % der Theorie.

### Beispiel 13

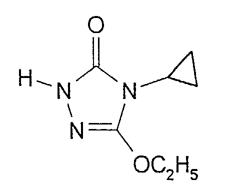

Analog zu Beispiel 2 erhält man durch Umsetzung von äquimolaren Mengen von Carbazinsäure-ethylester und Cyclopropyliminokohlensäure-0,0-diethylester in Gegenwart von Pivalinsäure (2 Mol-%) und anschließende Umsetzung des gebildeten Zwischenproduktes mit einer äquimolaren Menge NaOCH₃ 4-Cyclopropyl-5-ethoxy-2,4-dihydro-3H-1,2,4,-triazol-3-on(Ausbeute: 79 % d.Th.) vom Schmelzpunkt 144-145°C (umkristallisiert aus Wasser).

### Beispiel 14

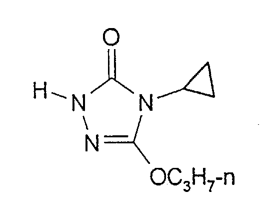

Analog zu den Beispielen 2 und 13, wobei jedoch anstelle des Carbazinsäureethylesters der Carbazinsäure-(2-ethoxyethylester) [vgl. Beispiel 12] eingesetzt wurde, erhält man 4-Cyclopropyl-5-propoxy-2,4-dihydro-3H-1,2,4-triazol-3-on (Ausbeute: 72 % d.Th.) vom Schmelzpunkt 105-106°C (aus Wasser).

### Beispiel 15

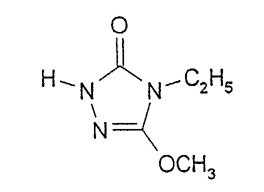

Analog zu den Beispielen 2 und 14 erhält man 4-Ethyl-5-methoxy-2,4-dihydro-3H-1,2,4-triazol-3-on (Ausbeute: 81 % d.Th.) vom Schmelzpunkt 130°C (aus Aceton).

### Zwischenprodukte der Formel (IV):

### Beispiel (IV-1)

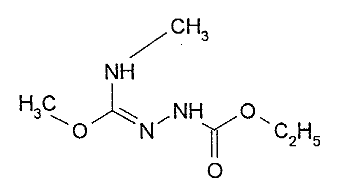

52 g (0,50 Mol) Carbazinsäure-ethylester werden in 400 ml Methanol vorgelegt und nach Zutropfen von 56,6 g (0,50 Mol) Methyliminokohlensäure-dimethylester (95%ig) wird die Mischung 16 Stunden bei 50°C und nach Zugabe von weiteren 8,2 g (0,08 Mol) Methyliminokohlensäure-dimethylester weitere 10 Stunden bei 50°C gerührt. Dann wird im Wasserstrahlvakuum eingeengt, der Rückstand mit Diethylether verrührt und das kristalline Produkt durch Absaugen isoliert.

Man erhält 75 g (86% der Theorie) N'-(α-Methylamino-α-methoxy-methylen)hydrazin-N-carbonsäure-ethylester vom Schmelzpunkt 128°C.

### Beispiel (IV-2)

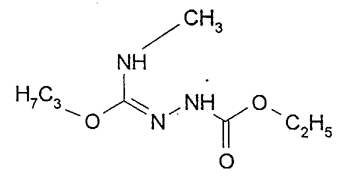

15,6 g (0,15 Mol) Carbazinsäure-ethylester werden in 100 ml n-Propanol vorgelegt und nach Zutropfen von 25,25 g (0,16 Mol) Methyliminokohlensäure-dipropylester wird die Mischung 16 Stunden bei 55°C bis 60°C und nach Zugabe von weiteren 4,7 g (0,03 Mol) Methyliminokohlensäure-dimethylester weitere 12 Stunden bei ca. 55°C gerührt. Dann wird im Wasserstrahlvakuum eingeengt, der Rückstand mit Methyl-t-butylether verrührt und das kristalline Produkt durch Absaugen isoliert.

Man erhält 24,65 g (81% der Theorie) N'-(α-Methylamino-α-propoxy-methylen)hydrazin-N-carbonsäure-ethylester vom Schmelzpunkt 104°C.

## Patentansprüche

1. Verfahren zur Herstellung von Alkoxytriazolinonen der allgemeinen Formel (I) 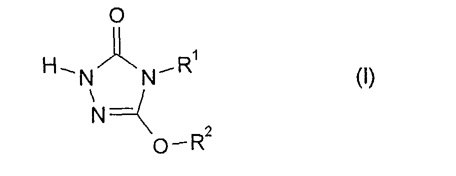 in welcher
R¹ für jeweils gegebenenfalls substituiertes Alkyl, Alkenyl, Alkinyl, Cycloalkyl, Cycloalkylalkyl, Aryl oder Arylalkyl steht und
R² für jeweils gegebenenfalls substituiertes Alkyl, Alkenyl, Alkinyl, Cycloalkyl, Cycloalkylalkyl, Aryl oder Arylalkyl steht,
dadurch gekennzeichnet, daß man Iminokohlensäurediester der allgemeinen Formel (II) 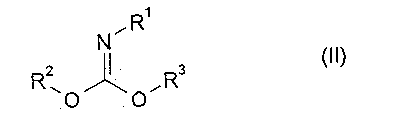 in welcher
R¹ und R² die oben angegebenen Bedeutungen haben und
R³ für jeweils gegebenenfalls substituiertes Alkyl, Aryl oder Arylalkyl steht,
mit Carbazinsäureestern der allgemeinen Formel (III), 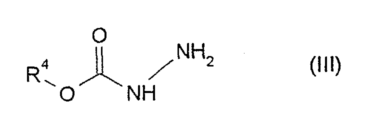 in welcher
R⁴ für jeweils gegebenenfalls substituiertes Alkyl, Aryl oder Arylalkyl steht,
gegebenenfalls in Gegenwart eines Reaktionshilfsmittels und gegebenenfalls in Gegenwart eines Verdünnungsmittels bei Temperaturen zwischen -20°C und +120°C umsetzt ("erste Umsetzungsstufe") und die hierbei gebildeten Semicarbazid-Derivate der allgemeinen Formel (IV), 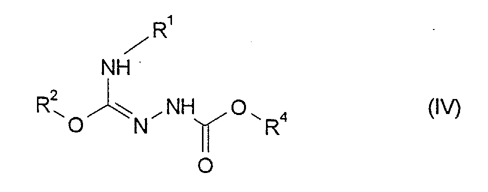 in welcher
R¹, R² und R⁴ die oben angegebenen Bedeutungen haben,
- und/oder die entsprechenden hierzu tautomeren Verbindungen -,
gegebenenfalls nach Zwischenisolierung, in Gegenwart einer Base und gegebenenfalls in Gegenwart eines Verdünnungsmittels bei Temperaturen zwischen 20°C und 100°C cyclisierend kondensiert ("zweite Umsetzungsstufe").

2. Verfahren nach Anspruch 1, dadurch gekennzeichnet, daß man die erste Umsetzungsstufe bei Temperaturen zwischen 0°C und 100°C, insbesondere zwischen 20°C und 80°C durchführt.

3. Verfahren nach Anspruch 1, dadurch gekennzeichnet, daß man die zweite Umsetzungsstufe bei Temperaturen zwischen 30°C und 90°C, insbesondere zwischen 40°C und 80°C durchführt.

4. Verfahren nach Anspruch 1, dadurch gekennzeichnet, daß Verbindungen der Formel (I) hergestellt werden, in welchen
R¹ für jeweils gegebenenfalls durch Halogen oder C₁-C₄-Alkoxy substituiertes Alkyl, Alkenyl oder Alkinyl mit jeweils bis zu 6 Kohlenstoffatomen, für jeweils gegebenenfalls durch Halogen oder C₁-C₄-Alkyl substituiertes Cycloalkyl oder Cycloalkylalkyl mit jeweils 3 bis 6 Kohlenstoffatomen im Cycloalkylteil und gegebenenfalls 1 bis 4 Kohlenstoffatomen im Alkylteil, oder für jeweils gegebenenfalls durch Carboxy, Cyano, Nitro, Halogen, C₁-C₄-Alkyl, C₁-C₄-Halogenalkyl, C₁-C₄-Alkoxy, C₁-C₄-Halogenalkoxy oder C₁-C₄-Alkoxy-carbonyl substituiertes Aryl oder Arylalkyl mit jeweils 6 oder 10 Kohlenstoffatomen im Arylteil und gegebenenfalls 1 bis 4 Kohlenstoffatomen im Alkylteil steht und
R² für jeweils gegebenenfalls durch Halogen oder C₁-C₄-Alkoxy substituiertes Alkyl, Alkenyl oder Alkinyl mit jeweils bis zu 6 Kohlenstoffatomen, für jeweils gegebenenfalls durch Halogen oder C₁-C₄-Alkyl substituiertes Cycloalkyl oder Cycloalkylalkyl mit jeweils 3 bis 6 Kohlenstoffatomen im Cycloalkylteil und gegebenenfalls 1 bis 4 Kohlenstoffatomen im Alkylteil, oder für jeweils gegebenenfalls durch Carboxy, Cyano, Nitro, Halogen, C₁-C₄-Alkyl, C₁-C₄-Halogenalkyl, C₁-C₄-Alkoxy, C₁-C₄-Halogenalkoxy oder C₁-C₄-Alkoxy-carbonyl substituiertes Aryl oder Arylalkyl mit jeweils 6 oder 10 Kohlenstoffatomen im Arylteil und gegebenenfalls 1 bis 4 Kohlenstoffatomen im Alkylteil steht.

5. Verfahren nach Anspruch 1, dadurch gekennzeichnet, daß als Iminokohlensäurediester der Formel (II) Methyliminokohlensäure-dimethylester, -diethylester, -dipropylester oder -O-(2-ethoxyethylester)-O-n-propylester eingesetzt wird.

6. Verfahren nach Anspruch 1, dadurch gekennzeichnet, daß als Carbazinsäureester der Formel (III) Carbazinsäure-ethylester eingesetzt wird.

7. Verfahren nach Anspruch 1, dadurch gekennzeichnet, daß als Verdünnungsmittel (in beiden Umsetzungsstufen) Alkohole, insbesondere Methanol, Ethanol, n- oder i-Propanol verwendet werden.

8. Verfahren nach Anspruch 1, dadurch gekennzeichnet, daß in der ersten Umsetzungsstufe als Reaktionshilfsmittel eine Protonensäure, insbesondere Pivalinsäure, Essigsäure oder (wäßrige) Salzsäure eingesetzt wird.

9. Verfahren nach Anspruch 1, dadurch gekennzeichnet, daß in der zweiten Umsetzungsstufe als Base eine organische oder anorganische Base, insbesondere ein Alkalimetallalkoholat oder -hydroxid - jeweils gegebenenfalls im entsprechenden Alkohol bzw. in Wasser gelöst - eingesetzt wird.

## Claims

1. Process for the preparation of alkoxytriazolinones of the general formula (I) 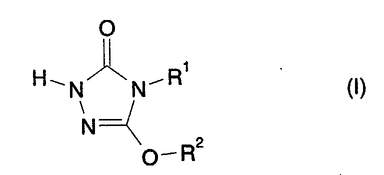 in which
R¹ represents in each case optionally substituted alkyl, alkenyl, alkinyl, cycloalkyl, cycloalkylalkyl, aryl or arylalkyl and
R² represents in each case optionally substituted alkyl, alkenyl, alkinyl, cycloalkyl, cycloalkylalkyl, aryl or arylalkyl,
characterized in that iminocarbonic diesters of the general formula (II) 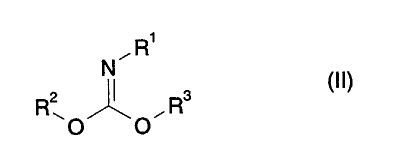 in which
R¹ and R² have the abovementioned meanings and
R³ represents in each case optionally substituted alkyl, aryl or arylalkyl,
are reacted with carbazinic esters of the general formula (III) 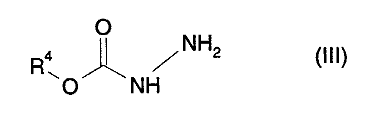 in which
R⁴ represents in each case optionally substituted alkyl, aryl or arylalkyl,
if appropriate in the presence of a reaction auxiliary and if appropriate in the presence of a diluent at temperatures between -20°C and +120°C ("first reaction step") and the semicarbazide derivatives formed in this process of the general formula (IV) 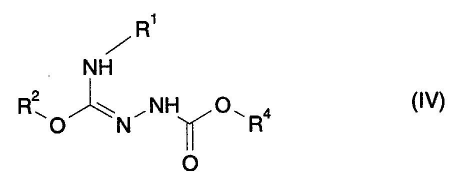 in which
R¹, R² and R⁴ have the abovementioned meanings,
- and/or the corresponding tautomeric compounds -
are subjected to a cyclizing condensation reaction, at temperatures between 20°C and 100°C, if appropriate after intermediate isolation, in the presence of a base and if appropriate in the presence of a diluent ("second reaction step").

2. Process according to Claim 1, characterized in that the first reaction step is carried out at temperatures between 0°C and 100°C, in particular between 20°C and 80°C.

3. Process according to Claim 1, characterized in that the second reaction step is carried out at temperatures between 30°C and 90°C, in particular between 40°C and 80°C.

4. Process according to Claim 1, characterized in that compounds of the formula (I) are prepared in which
R¹ represents alkyl, alkenyl or alkinyl, each of which has up to 6 carbon atoms and each of which is optionally substituted by halogen or C₁-C₄-alkoxy, or represents cycloalkyl or cycloalkylalkyl, each of which has 3 to 6 carbon atoms in the cycloalkyl moiety and, if appropriate, 1 to 4 carbon atoms in the alkyl moiety and each of which is optionally substituted by halogen or C₁-C₄-alkyl, or represents aryl or arylalkyl, each of which has 6 or 10 carbon atoms in the aryl moiety and, if appropriate, 1 to 4 carbon atoms in the alkyl moiety and each of which is optionally substituted by carboxyl, cyano, nitro, halogen, C₁-C₄-alkyl, C₁-C₄-halogenoalkyl, C₁-C₄-alkoxy, C₁-C₄-halogenoalkoxy or C₁-C₄-alkoxy-carbonyl, and
R² represents alkyl, alkenyl or alkinyl, each of which has up to 6 carbon atoms and each of which is optionally substituted by halogen or C₁-C₄-alkoxy, or represents cycloalkyl or cycloalkylalkyl, each of which has 3 to 6 carbon atoms in the cycloalkyl moiety and, if appropriate, 1 to 4 carbon atoms in the alkyl moiety and each of which is optionally substituted by halogen or C₁-C₄-alkyl, or represents aryl or arylalkyl, each of which has 6 or 10 carbon atoms in the aryl moiety and, if appropriate, 1 to 4 carbon atoms in the alkyl moiety and each of which is optionally substituted by carboxyl, cyano, nitro, halogen, C₁-C₄-alkyl, C₁-C₄-halogenoalkyl, C₁-C₄-alkoxy, C₁-C₄-halogenoalkoxy or C₁-C₄-alkoxy-carbonyl.

5. Process according to Claim 1, characterized in that the iminocarbonic diester of the formula (II) employed is dimethyl methyliminocarbonate, diethyl methyliminocarbonate, dipropyl methyliminocarbonate or O-(2-ethoxyethyl) O-n-propyl methyliminocarbonate.

6. Process according to Claim 1, characterized in that the carbazinic ester of the formula (III) employed is ethyl carbazinate.

7. Process according to Claim 1, characterized in that the diluents used (in both reaction steps) are alcohols, in particular methanol, ethanol or n- or i-propanol.

8. Process according to Claim 1, characterized in that the reaction auxiliary employed in the first reaction step is a protonic acid, in particular pivalic acid, acetic acid or (aqueous) hydrochloric acid.

9. Process according to Claim 1, characterized in that the base employed in the second reaction step is an organic or inorganic base, in particular an alkali metal alcoholate or alkali metal hydroxide, in each case dissolved, if appropriate, in an appropriate alcohol or in water.

## Revendications

1. Procédé de production d'alkoxytriazolinones de formule générale (I) 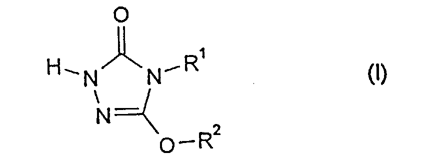 dans laquelle
R¹ représente un groupe alkyle, alcényle, alcynyle, cycloalkyle, cycloalkylalkyle, aryle ou arylalkyle, chacun étant éventuellement substitué, et
R² est un groupe alkyle, alcényle, alcynyle, cycloalkyle, cycloalkylalkyle, aryle ou arylalkyle, chacun étant éventuellement substitué,
caractérisé en ce qu'on fait réagir des diesters d'acides iminocarboniques de formule générale (II) 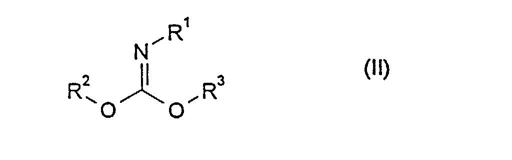 dans laquelle
R¹ et R² ont les définitions indiquées ci-dessus, et
R³ représente un groupe alkyle, aryle ou arylalkyle dont chacun est éventuellement substitué,
avec des esters d'acide carbazique de formule générale (III) 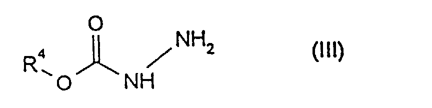 dans laquelle
R⁴ est un groupe alkyle, aryle ou arylalkyle, dont chacun est éventuellement substitué,
le cas échéant en présence d'un auxiliaire de réaction et en la présence éventuelle d'un diluant, à des températures comprises entre -20°C et +120°C ("premier stade de réaction") et on condense par cyclisation ("second stade de réaction") les dérivés de semicarbazide ainsi formés de formule générale (IV) 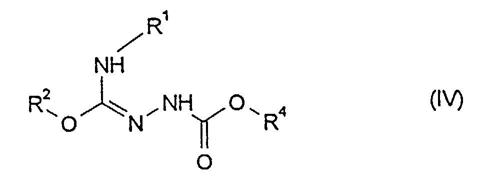 dans laquelle
R¹, R² et R⁴ ont les définitions indiquées ci-dessus,
- et/ou les composés tautomères qui y correspondent -, le cas échéant après isolement intermédiaire, en présence d'une base et en la présence éventuelle d'un diluant, à des températures comprises entre 20°C et 100°C.

2. Procédé suivant la revendication 1, caractérisé en ce qu'on conduit le premier stade de réaction à des températures comprises entre 0°C et 100°C, notamment entre 20°C et 80°C.

3. Procédé suivant la revendication 1, caractérisé en ce qu'on conduit le second stade de réaction à des températures comprises entre 30°C et 90°C, notamment entre 40°C et 80°C.

4. Procédé suivant la revendication 1, caractérisé en ce qu'il produit des composés de formule (I) dans lesquels
R¹ représente un groupe alkyle, alcényle ou alcynyle ayant dans chaque cas jusqu'à 6 atomes de carbone et substitué chacun le cas échéant par un halogène ou un groupe alkoxy en C₁ à C₄, un groupe cycloalkyle ou cycloalkylalkyle ayant chacun 3 à 6 atomes de carbone dans la partie cycloalkyle et le cas échéant 1 à 4 atomes de carbone dans la partie alkyle et étant chacun substitué le cas échéant par un halogène ou un groupe alkyle en C₁ à C₄, ou bien un groupe aryle ou arylalkyle ayant chacun 6 ou 10 atomes de carbone dans la partie aryle et le cas échéant 1 à 4 atomes de carbone dans la partie alkyle et chacun étant substitué le cas échéant par un groupe carboxy, cyano, nitro, halogéno, alkyle en C₁ à C₄, halogénalkyle en C₁ à C₄, alkoxy en C₁ à C₄, halogénalkoxy en C₁ à C₄ ou (alkoxy en C₁ à C₄)-carbonyle et
R² représente un groupe alkyle, alcényle ou alcynyle ayant dans chaque cas jusqu'à 6 atomes de carbone et chacun étant substitué le cas échéant par un halogène ou un groupe alkoxy en C₁ à C₄, un groupe cycloalkyle ou cycloalkylalkyle ayant dans chaque cas 3 à 6 atomes de carbone dans la partie cycloalkyle et le cas échéant 1 à 4 atomes de carbone dans la partie alkyle et chacun étant substitué le cas échéant par un halogène ou un groupe alkyle en C₁ à C₄, ou bien un groupe aryle ou arylalkyle ayant dans chaque cas 6 ou 10 atomes de carbone dans la partie aryle et le cas échéant 1 à 4 atomes de carbone dans la partie alkyle et portant chacun le cas échéant un substituant carboxy, cyano, nitro, halogéno, alkyle en C₁ à C₄, halogénalkyle en C₁ à C₄, alkoxy en C₁ à C₄, halogénalkoxy en C₁ à C₄ ou (alkoxy en C₁ à C₄)-carbonyle.

5. Procédé suivant la revendication 1, caractérisé en ce qu'on utilise comme diesters d'acides iminocarboniques de formule (II), l'ester diméthylique, l'ester diéthylique, l'ester dipropylique ou l'ester de O-(2-éthoxyéthyle) et O-(n-propyle) de l'acide méthylimino-carbonique.

6. Procédé suivant la revendication 1, caractérisé en ce qu'on utilise comme ester d'acide carbazique de formule (III) l'ester éthylique de l'acide carbazique.

7. Procédé suivant la revendication 1, caractérisé en ce qu'on utilise comme diluants (dans les deux stades réactionnels) des alcools, en particulier le méthanol, l'éthanol, le n-propanol et l'isopropanol.

8. Procédé suivant la revendication 1, caractérisé en ce qu'on utilise comme auxiliaire de réaction dans le premier stade de réaction un acide protonique, en particulier l'acide pivalique, l'acide acétique ou l'acide chlorhydrique (aqueux).

9. Procédé suivant la revendication 1, caractérisé en ce qu'on utilise comme base dans le second stade de réaction une base organique ou inorganique, en particulier un alcoolate ou hydroxyde de métal alcalin, éventuellement dissous à chaque fois dans l'alcool correspondant ou dans l'eau.
